# EUROPÄISCHE PATENTSCHRIFT

(11) **EP 1 563 743 B1**
(45) Veröffentlichungstag und Bekanntmachung des Hinweises auf die Patenterteilung: **16.04.2008**
(21) Anmeldenummer: 05008835.0
(22) Anmeldetag: 07.08.2002
(51) Int. Cl.: A23L 1/30, A23L 1/302

(54) **Mischung enthaltend eine koffeinhaltige Substanz, insbesondere Guarana**
Composition comprising a caffeine-containing substance, particularly guarana
Composition comprenant une substance contenant de la caféine, en particulier guarana

(30) Priorität: 13.09.2001 DE 10145246
(43) Veröffentlichungstag der Anmeldung: 17.08.2005
(62) Teilanmeldung aus: 02017559.2
(73) Patentinhaber: PM-International AG, 1618 Luxembourg (LU)
(72) Erfinder: Schmitt, Gerhard, Dr., 65625 Bensheim (DE); Schwietz, Horst, Dr., 90584 Allersberg (DE)
(74) Vertreter: Zech, Stefan Markus

(56) Entgegenhaltungen:
- EP-A- 0 916 269
- GB-A- 2 285 578

## Beschreibung

Die Erfindung betrifft eine Mischung enthaltend eine koffeinhaltige Substanz, insbesondere Guarana, Niacin und B-Vitamine, sowie mindestens eine als Radikalfänger wirkende Substanz, sowie getrocknete Braunalgen, wahlweise in Form von dosen Salzen.

Eine derartige Mischung ist bereits bekannt und wird auf dem Markt als Nahrungsergänzungspräparat angeboten.

Die vorgenannte Mischung kann einige positive, gewünschte Wirkungen bei Verabreichung in angemessener Dosis erzielen, wie beispielsweise Erhöhung der körperlichen und geistigen Leistungsbereitschaft, Förderung der Durchblutung, etc..

In EP 0 916 269 A1 wird ein Nahrungsmittel oder Getränk beschrieben, das Fucoidan enthält, wobei das Fucoidan aus einer Fucoidan-haltigen Substanz deriviert worden ist. Des Weiteren werden dort viele verschiedene Rezepturen für ein solches erfindungsgemäßes Nahrungsmittel oder Getränk beschrieben, wobei in dessen Beispiel 7 in der zugehörigen Tabelle 5 (Seite 21) eine Zusammensetzung für einen Nährdrink ("nutritive drink") mit u. a. folgenden Bestandteilen explizit angegeben wird: "Guarana extract", "Vitamin C", "Nicotin-amide", "Vitamin B₁ hypochloride" und "Vitamin B₆ hypochloride". Außerdem wird in der zum Beispiel 7 gehörigen Beschreibung die Zugabe von Fucoidan genannt.

In GB 2 285 578 A werden koffeinhaltige und koffeinfreie Getränke z. B. Tee, Schokolade oder Cola-Drinks offenbart, die zugesetztes Guarana enthalten. Das Guarana wird dabei in reiner Form oder als Samen oder Extrakte der sog. "Paulina Cupana"-Pflanze zugefügt, wobei außerdem zusätzliche Vitamine oder Kräuterextrakte zugegeben werden können. Der Konsument eines solchen Getränkes gemäß GB 2 285 578 A spürt dadurch eine größere Energie, Vitalität, Wachheit usw..

Die Aufgabe der vorliegenden Erfindung besteht darin, die Wirkungen eines vorwiegend, aber nicht unbedingt ausschließlich als Nahrungsergänzungsmittel konzipierten Präparates der oben angegebenen Art noch zu verbessern.

Diese Aufgabe wird mit einer Mischung nach den Merkmalen des Anspruches 1 gelöst. Ein Kerngedanke der Erfindung liegt dabei darin, dass in synergistischer Weise eine koffeinhaltige Substanz, insbesondere Guarana, Niacin, B-Vitamine, eine als Radikalfänger wirkende Substanz sowie Braunalgen, wahlweise in Form der Salze miteinander kombiniert werden.

Die erfindungsgemäße Mischung besitzt gute Sauerstofftransporteigenschaften und zeichnet sich weiter dadurch aus, dass Zellbarrieren hervorragend durchdrungen werden und so Sauerstoff nicht nur transportiert, sondern den Zellen selbst zusätzlich Sauerstoff zugeführt wird. Die Algensubstanz umfassend getrocknete Braunalgen verstärkt in synergistischer Weise die Wirkung der weiterhin enthaltenen Substanzen. Guarana als koffeinhaltige Substanz wird bevorzugt, da es eine im Vergleich zu Koffein längere Wirkungszeit aufweist und damit der erwünschte Wachhalteeffekt länger andauert. Als Radikalfänger wirkende Substanzen kommen antioxidativ wirkende Vitamine, wie beispielsweise Vitamin C in Betracht.

In einem bevorzugten Ausführungsbeispiel beträgt der Mindestgewichtsanteil der getrockneten Braunalgen insgesamt mind. 0.0001 Gew. % bezogen auf die durch die oben angegebenen Inhaltsstoffe definiert Mischung.

Weiter vorteilhaft liegt die Konzentration getrockneter Braunalgen insgesamt in einem Bereich zwischen 0.0001 und 2 Gew. % bezogen auf die durch die obigen Substanzen definierte Mischung. Die o. g. Konzentrationsangabe der getrockneten Braunalgen erfolgte allein bezogen auf die zuvor angegebenen Substanzen. Die erfindungsgemäße Mischung enthält vorteilhafterweise aber auch zu einem hohen Anteil noch Zusatzstoffe, wie Träger- oder Geschmacksstoffe. Die Trägerstoffe können auch eine Resorptionsverbesserung der Mischung im Körper bewirken. Die Konzentration dieser Zusatzstoffe liegt vorteilhafterweise im Bereich zwischen 20 und 95 Gew. % der Mischung, weiter vorzugsweise im Bereich zwischen 60 und 95 Gew. %.

In der hier bevorzugten Ausführungsform liegt die Mischung in pulvriger Form zur Herstellung einer wässrigen Lösung vor, wobei eine Dosierung von 1 bis 20 g auf ein Glas (0,2 1 bis 1,0 1) Wasser zweckmäßig erscheint.

Alternativ kann die Mischung aber auch in flüssiger oder fester Form, beispielsweise als Tablette, vorliegen.

Die erfindungsgemäße Mischung kann als Mittel zur Erhöhung/Stimulation des O₂-Antransportes in menschlichen oder tierischen Zellsystemen verwendet werden. Durch die synergistische Wirkung der getrockneten Braunalgen mit der koffeinhaltigen Substanz sowie den weiteren enthaltenden Substanzen wird den Zellen wesentlich mehr Sauerstoff zugeführt, was sich in konkreten Experimenten in Steigerung der körperlichen und geistigen Leistungsfähigkeiten nachweisen ließ.

Eine weitere Wirkung der erfindungsgemäßen Mischung besteht darin, dass die Durchblutung ganz merklich gefördert wird. Die Wirkung der Mischung selbst setzt nach oraler Einnahme in etwa nach 10 Minuten ein, wird von den Probanden als Wärmesensation auf bestimmten Hautpartien empfunden und lässt sich mit thermografischen Bildaufnahmeverfahren, insbesondere in einer thermografischen Bildserie belegen. Die thermografischen Untersuchungen belegen, dass die Wirkungen der erfindungsgemäßen Mischung auf einige Körperpartien einschließlich des Kopfes erstrecken.

In einer ersten Studie konnte belegt werden, dass die erfindungsgemäße Mischung, wie bereits zuvor eine der Einzelkomponenten dieser Mischung, die einer Stichprobe von Probanden oral verabreicht wurde, einen signifikaten Einfluss auf die körperliche Leistungsfähigkeit ausübt, was sich an der signifikanten Erhöhung des RQ's (Respiratorischer Quotient) bestätigen ließ.

Ein Reaktionstest zeigte, dass die Vigilanz der Probanden durch die erfindungsgemäße Mischung gesteigert werden konnte.

## Patentansprüche

1. Mischung enthaltend
- eine koffeinhaltige Substanz, insbesondere Guarana,
- Niacin und andere B-Vitamine,
- mindestens eine als Radikalfänger wirkende Substanz sowie
- getrocknete Braunalgen, wahlweise in Form von deren Salzen.

2. Mischung nach Anspruch 1,
**dadurch gekennzeichnet,**
**dass** getrocknete Braunalgen insgesamt in einer Konzentration von mindestens 0.0001 Gew. % enthalten sind.

3. Mischung nach einem der Ansprüche 1 bis 2,
**dadurch gekennzeichnet,**
**dass** getrocknete Braunalgen insgesamt in einer Konzentration zwischen 0.0001 und 2 Gew. % enthalten sind.

4. Mischung nach einem der Ansprüche 1 bis 3,
**dadurch gekennzeichnet,**
**dass** die Mischung weiterhin Zuschläge, wie Träger- oder Geschmacksstoffe enthält.

5. Mischung nach Anspruch 4,
**dadurch gekennzeichnet,**
**dass** der Gewichtsanteil der Zuschläge an der Mischung im Bereich zwischen 20 und 95 Gew. %, vorzugsweise im Bereich zwischen 60 und 95 Gew. % liegt.

6. Mischung nach einem der Ansprüche 1 bis 5,
**dadurch gekennzeichnet,**
**dass** die Mischung in pulvriger Form zur Herstellung einer wässrigen Lösung vorliegt.

7. Mischung nach einem der Ansprüche 1 bis 5,
**dadurch gekennzeichnet,**
**dass** die Mischung in fester oder flüssiger Form vorliegt.

8. Verwendung der Mischung nach einem der Ansprüche 1 bis 7 zur Herstellung eines Mittel zur Erhöhung/Stimulation des O₂-Antransportes in menschliche oder tierische Zellsysteme.

9. Verwendung der Mischung nach einem der Ansprüche 1 bis 7 zur Herstellung eines Mittels zur Förderung der Durchblutung.

10. Verwendung der Mischung nach einem der Ansprüche 1 bis 7 als ein die Konzentration förderndes Mittel.

## Claims

1. Mixture comprising
- a caffeine-containing substance, particularly guarana,
- niacin and other B vitamins,
- at least one substance which acts as an agent which traps free radicals and
- dried brown algae, optionally in the form of salts thereof.

2. Mixture according to claim 1,
**characterized in that**
it comprises the dried brown algae in total in a concentration of at least 0.0001 wt.%.

3. Mixture according to one of claims 1 to 2,
**characterized in that**
it comprises the dried brown algae in total in a concentration of between 0.0001 and 2 wt.%.

4. Mixture according to one of claims 1 to 3,
**characterized in that**
the mixture furthermore comprises additives, such as carrier or flavouring substances.

5. Mixture according to claim 4,
**characterized in that**
the proportion by weight of the additives in the mixture is in the range between 20 and 95 wt.%, preferably in the range between 60 and 95 wt.%.

6. Mixture according to one of claims 1 to 5,
**characterized in that**
the mixture is in a powdery form for the preparation of an aqueous solution

7. Mixture according to one of claims 1 to 5,
**characterized in that**
the mixture is in a solid or liquid form.

8. Use of the mixture according to one of claims 1 to 7 for the preparation of an agent for increasing/stimulating the transportation of O₂ into human or animal cell systems.

9. Use of the mixture according to one of claims 1 to 7 for the preparation of an agent for promoting blood circulation.

10. Use of the mixture according to one of claims 1 to 7 as a concentration-promoting agent.

## Revendications

1. Mélange contenant
une substance contenant de la caféine, en particulier, du guarana,
de la nyacine et autres Vitamines B
au moins une substance servant à séquestrer les radicaux ainsi que des extraits d'algues brunes séchées, éventuellement sous la forme de leurs sels.

2. Mélange selon la revendication 1,
**caractérisé**
**en ce que** les extraits d'algues brunes séchés sont contenus dans l'ensemble à une concentration d'au moins 0,0001% pds.

3. Mélange selon l'une quelconque des revendications 1 à 2,
**caractérisé**
**en ce que** les extraits d'algues brunes séchés sont contenus dans l'ensemble à une concentration comprise entre 0,0001 et 2% pds.

4. Mélange selon l'une quelconque des revendications 1 à 3,
**caractérisé**
**en ce que** le mélange contient de plus des additifs comme des supports ou des saveurs.

5. Mélange selon la revendication 4,
**caractérisé**
**en ce que** la proportion pondérale des additifs au mélange est comprise entre 20 et 95% pds, avantageusement entre 60 et 95% pds.

6. Mélange selon l'une quelconque des revendications 1 à 5,
**caractérisé**
**en ce que** le mélange est sous forme pulvérisée pour la production d'une solution dans l'eau.

7. Mélange selon l'une quelconque des revendications 1 à 5,
**caractérisé**
**en ce que** le mélange est sous une forme solide ou liquide.

8. Mélange selon l'une quelconque des revendications 1 à 7, pour la production d'un moyen pour l'augmentation/stimulation du transport de O₂ dans les systèmes cellulaires humain ou animaux.

9. Utilisation du mélange selon l'une quelconque des revendications 1 à 7, pour la fabrication d'un agent améliorant la circulation sanguine.

10. Utilisation du mélange selon l'une quelconque des revendications 1 à 7 pour une utilisation en tant qu'agent améliorant la concentration.
